# EUROPÄISCHE PATENTSCHRIFT

(11) **EP 0 401 744 B1**
(45) Veröffentlichungstag und Bekanntmachung des Hinweises auf die Patenterteilung: **30.11.1994**
(21) Anmeldenummer: 90110607.0
(22) Anmeldetag: 05.06.1990
(51) Int. Cl.: A61F 13/36, A61F 15/00, A61B 19/02

(54) **Packung zum Bereithalten von Tupfern**
Package for handy storage of swabs
Emballage pour stockage des tampons chirurgicaux

(30) Priorität: 05.06.1989 DE 8906888 U
(43) Veröffentlichungstag der Anmeldung: 12.12.1990
(73) Patentinhaber: RAUSCHER & CO., VERBANDSTOFF- UND WATTEFABRIKEN GES.M.B.H., A-2525 Schönau/Tr. (AT)
(72) Erfinder: Leuprecht, Helmut, Dr., A-1130 Wien (AT)
(74) Vertreter: Patentanwälte Leinweber & Zimmermann

(56) Entgegenhaltungen:
- FR-A- 509 998
- FR-A- 2 426 447
- GB-A- 2 112 755
- US-A- 4 046 254

## Beschreibung

Die Erfindung betrifft eine Einrichtung zum Bereithalten chirurgischer Hilfsmittel mit einer Vielzahl von vorzugsweise in mehreren Reihen angeordneten separaten Aufnahmen für je ein chirurgisches Hilfsmittel.

Derartige Einrichtungen zum Bereithalten chirurgischer Hilfsmittel mit einer Vielzahl von vorzugsweise in mehreren Reihen angeordneten separaten Aufnahmen sind aus der US-A-4,046,254 bereits bekannt. Die bekannte Einrichtung ist eine Aufnahmeschale für chirurgische Instrumente. Für je ein chirurgisches Instrument, wie ein Skalpell oder eine Schere, ist eine Aufnahme vorgesehen. Die Aufnahmen sind in zwei Reihen angeordnet. Vor einer Operation werden die einzelnen Aufnahmen je nach den Bedürfnissen der bevorstehenden Operation auf eine "programmierte" Weise befüllt, so daß die chirurgischen Instrumente in der bei der Operation benötigten richtigen Reihenfolge bereitliegen. Hierzu werden entsprechende Aufnahmekarten verfertigt. Die Aufnahmekarte erlaubt es auch, nicht nur die Instrumentenschale der Operation entsprechend zu befüllen, sondern auch während der Operation sogleich zu erkennen, welches Instrument in einer leer gebliebenen Aufnahme fehlt. Die in der Instrumentenschale entsprechend der Aufnahmekarte bereitgelegten Instrumente können in ihrer Lage durch einen auf die Instrumentenschale aufgedrückten Deckel fixiert und in dieser Stellung sterilisiert werden. Alle vorbeschriebenen Arbeiten erfolgen im Operationssaal. Eine Handelspackung der Instrumente stellt die Instrumentenschale natürlich nicht dar.

Bekannt ist weiter aus der GB-A-2,112,755 eine Aufnahmeschale für menschliches Gewebe für Zwecke der Autopsie von Leichen. Die Einteilung in völlig unterschiedlich geformte Aufnahmen soll dabei sicherstellen, daß tatsächlich in jedem Einzelfall alle benötigten Gewebeteile entnommen werden. Durch das Einlegen in das für jedes spezifische Gewebeteil vorgesehene Fach wird überdies die spätere Zuordnung der unterschiedlichen Gewebeteile durch die Einordnung in die unterschiedlichen Fächer unterstützt.

Die bei Operationen benötigten Tupfer, insbesondere Kugeltupfer und Präpariertupfer werden bisher in abgezählter Anzahl von zehn oder zwanzig Tupfern steril in Plastiksäckchen bereitgehalten. Um zu vermeiden, daß derartige Tupfer im Operationsfeld und damit im Körper belassen werden, wird bisher so vorgegangen, daß die in fester Anzahl vorhandenen Tupfer durch Durchzählen darauf überprüft werden, ob alle Tupfer wieder aus dem Operationsfeld rückgeführt wurden.

Aufgabe der Erfindung ist es, die Sicherheit dadurch zu erhöhen und das OP-Personal so zu entlasten, daß ohne zusätzliche Arbeitsgänge und ohne Zählen der vollständige Rücklauf aller Tupfer gesichert wird.

Diese Aufgabe wird erfindungsgemäß dadurch gelöst, daß eine Tupferpackung mit einer Vielzahl von separaten Ein-Tupfer-Aufnahmen vorgesehen wird, von denen jede mit einem einzigen Tupfer befüllt ist, wobei die befüllte und sterilisierte Tupferpackung in eine Plastikhülle eingeschlossen ist.

Wurden bisher die sterilisierten Tupfer einfach lose in einem Plastikbeutel gehandelt, so tritt hier eine zusätzliche, die Tupfer in der Plastikhülle halternde Tupferpackung hinzu. Diese weist für jeden Tupfer eine separate Ein-Tupfer-Aufnahme auf. Ihre Größe ist also so vorgesehen, daß jede der Ein-Tupfer-Aufnahmen zwar gerade einen Tupfer, nicht aber zwei Tupfer aufnehmen kann. Dadurch wird zwangsweise erreicht, daß aus dem Operationsfeld rücklaufende Tupfer wieder je für sich in eine der separaten Aufnahmen rückgelegt werden. Überdies kann der Arzt oder Operationschef jederzeit mit einem Blick feststellen, ob bereits sämtliche Tupfer aus dem Operationsfeld in die Tupferpackung zurückgeführt wurden oder noch nach einem fehlenden Tupfer gesucht werden muß. Der bisher erforderliche Zählarbeitsgang entfällt. Die Kontrolle ist mit einfachen Mitteln gesteigert, die Sicherheit erhöht. Ein zusätzlicher Arbeitsgang ist nicht notwendig. Die Handhabung ist im Gegenteil vereinfacht. Denn sämtliche Tupfer können mit einem Handgriff in der sterilisierten Tupferpackung aus der Plastikhülle entnommen und für die Operation bereitgestellt werden.

Um die Tupfer in der unterteilten Tupferpackung sicher zu haltern, aber auch sicher voneinander zu trennen, ist eine Abstimmung der in der Tupferpackung ausgebildeten Aufnahmemulden auf die jeweilige Tupfergröße zweckmäßig. So soll der Durchmesser oder die große Diagonale der Aufnahmemulde nur geringfügig größer sein als die größte Länge des einzulegenden Tupfers, so daß der Charakter als Ein-Tupfer-Aufnahme gesichert und keinesfalls wegen Übergröße das Einlegen von zwei Tupfern in ein und dieselbe Aufnahme möglich ist. Gleichzeitig wird zweckmäßig die Tiefe der Aufnahmemulde größer als die kleinste Querabmessung des Tupfers gewählt, so daß der Tupfer vollständig in die Aufnahmemulde aufgenommen wird und nicht über deren oberen Rand vorsteht.

Selbstverständlich können die einzeln in die Aufnahmemulde der Tupferpackung eingelegten Tupfer durch entsprechend knappe Ausbildung der Plastikhülle in ihren separaten Aufnahmen gehalten werden. Noch einfacher ist es aber, die Tupferpackung zweiteilig auszubilden und dabei die Aufnahmemulde in einem Hauptteil vorzusehen, dem ein als Schiebehülse ausgebildeter Deckel zugeordnet ist.

Zwei Ausführungsformen der Erfingung werden anhand der Figuren erläutert und zwar zeigen
Fig. 1 perspektivisch eine erste Ausführungsform des Hauptteils mit Ein-Tupfer-Aufnahmemulden befüllt mit zwanzig Tupfern, und
Fig. 2 perspektivisch eine weitere Ausführungsform mit einer zweiteiligen Tupferpackung mit teilweise aus der Schiebehülse herausgezogenem Hauptteil.

Die Figuren zeigen zur besseren Übersicht die sterilisierten Tupferpackungen nach der Entnahme aus der Plastikhülle, in der sie gehandelt beziehungsweise gehandhabt werden. In den gezeigten Ausführungsbeispielen besteht die Tupferpackung aus einer Karton-Faltschachtel.

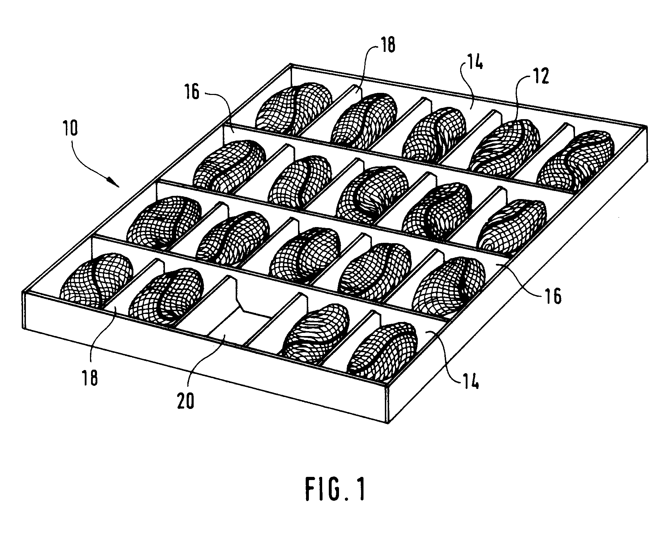

Fig. 1 zeigt in dem umrißquadratischen Hauptteil eine Tupferpackung 10 für zwanzig Präpariertupfer 12. Diese sind in Ein-Tupfer-Aufnahmemulden 14 vorgesehen, wobei in jeder Reihe fünf Tupfer untergebracht sind. Wegen der quadratischen Gesamtabmessung der Tupferpackung 10 sind also die Aufnahmemulden rechteckig. Sie weisen eine geringere Breite als Länge auf. Das entspricht der Form der Präpariertupfer 12, die eine gegenüber ihrer Länge geringere Querabmessung haben. Die separaten Ein-Tupfer-Aufnahmemulden 14 werden durch drei Querstege 16 und vier Längsstege 18 voneinander getrennt. Auch die Quer- und Längsstege bestehen aus Karton. Sie sind untereinander durch entsprechende Einschnitte verbunden.

Die Präpariertupfer 12 liegen also auf dem Boden 20 der Tupferpackung 10 auf und werden so je für sich in eine Ein-Tupfer-Aufnahmemulde 14 aufgenommen, wobei benachbarte Tupfer voneinander durch die Querstege 16 beziehungsweise die Längsstege 18 getrennt sind.

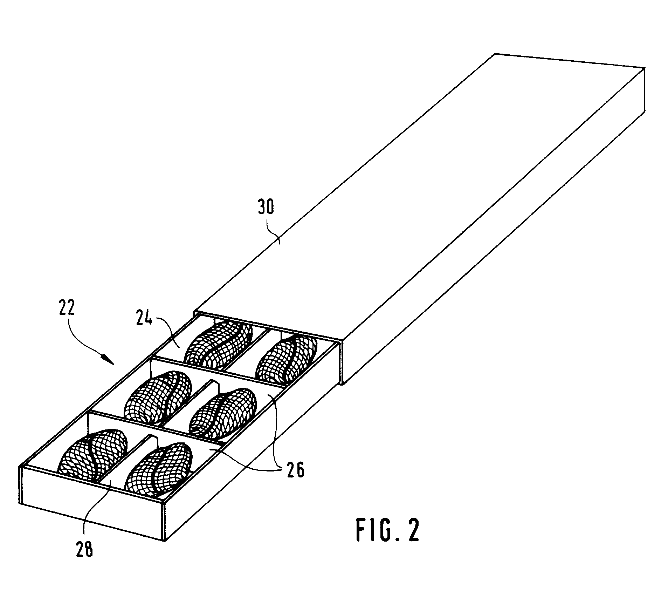

Fig. 2 zeigt eine weitere Ausführungsform einer Packung für zehn Präpariertupfer 12, die in zwei Reihen von fünf Tupfern angeordnet sind. Durch entsprechende Wahl der Abmessungen des Hauptteils 22 (Seitenlängenverhältnis beispielsweise 1,8) werden auch hier wieder rechteckige Ein-Tupfer-Aufnahmemulden 24 einer der länglichen Form der Präpariertupfer 12 entsprechenden Form erzielt. Während bei der Ausführungsform nach Fig. 1 drei Querstege 16 und vier Längsstege 18 vorhanden sind, gibt es hier vier Querstege 26 und nur noch einen Längssteg 28.

Die Ausführungsform von Fig. 2 unterscheidet sich aber von derjenigen nach Fig. 1 zusätzlich durch einen Deckel in Form einer Schiebehülse 30, die ebenfalls aus Karton besteht und einfach über den Hauptteil 22 geschoben wird. Dadurch werden die einzelnen Präpariertupfer 12 in ihren Ein-Tupfer-Aufnahmemulden 24 fixiert.

Entscheidend ist, daß eine vorgegebene Anzahl von Präpariertupfern (beispielsweise zehn oder zwanzig) in einer ebenen Fläche auf den ersten Blick übersichtlich angeordnet sind. Die Tupfer liegen also nicht mehr lose in einem Säckchen, sondern je für sich in einer Ein-Tupfer-Aufnahmemulde einer zusätzlichen Tupferpackung. Aus dieser können die Tupfer einfach entnommen und ebenso einfach je für sich in ihre separate Ein-Tupfer-Aufnahmemulde zurückgelegt werden. Mit einem Blick kann kontrolliert werden, ob eine Aufnahmemulde unbesetzt, ein Präpariertupfer 12 also im Operationsbereich verblieben ist.

Die zusätzliche Kontrolle der in der Tupferpackung 10 und mit dieser sterilisierten Präpariertupfer 12 erfolgt beispielsweise wie folgt: Bei der Entnahme aus der Plastikhülle werden an der Tupferpackung zwei Zettel angebracht, von denen im OP einen die die Tupferpackung übergebende Schwester, den anderen die steril arbeitende Operationsschwester erhält. Beide Zettel müssen bei der Rückgabe der Packung mit den zumindest teilweise gebrauchten Präpariertupfern wieder zusammengefügt werden und bestätigen, daß die Packung wiederum alle Präpariertupfer enthält, so daß kein Präpariertupfer im Operationsfeld vergessen werden kann.

## Patentansprüche

1. Einrichtung zum Bereithalten chirurgischer Hilfsmittel mit einer Vielzahl von, vorzugsweise in mehreren Reihen angeordneten, separaten Aufnahmen für je ein chirurgisches Hilfsmittel, dadurch gekennzeichnet,
daß es sich bei dem chirurgischen Hilfsmittel um Tupfer (12) und bei der Einrichtung um eine Tupferpackung (10) handelt,
daß von den separaten Aufnahmen jede als Ein-Tupfer-Aufnahme (14) ausgebildet und mit einem einzigen Tupfer (12) befüllt ist,
und daß die befüllte und sterilisierte Tupferpackung (10) in einer Plastikhülle eingeschlossen ist.

2. Einrichtung nach Anspruch 1, dadurch gekennzeichnet, daß die Ein-Tupfer-Aufnahmen (14, 24) muldenförmig ausgebildet sind, wobei der Durchmesser oder die große Diagonale der Aufnahmenmulde größer ist als die größte Länge des einzulegenden Tupfers (12) und die Tiefe größer ist als die kleinste Querabmessung des Tupfers (12).

3. Einrichtung nach Anspruch 1 oder 2, dadurch gekennzeichnet, daß die Tupferpackung (10) zweiteilig aus Hauptteil (22) und einem als Schiebehülse (30) gebildeten Dekkel besteht.

## Claims

1. Device for keeping surgical aids at the ready, having a multiplicity of separate receptacles which are preferably arranged in several rows and are each intended for a surgical aid, characterized
in that the surgical aid is a swab (12) and the device is a swab package (10),
in that each of the separate receptacles is designed as a single-swab receptacle (14) and is filled with a single swab (12),
and in that the filled and sterilized swab package (10) is enclosed in a plastic covering.

2. Device according to Claim 1, characterized in that the single-swab receptacles (14, 24) are of trough-shaped design, the diameter or the large diagonal of the receptacle trough being greater than the maximum length of the swab (12) to be inserted, and the depth being greater than the smallest transverse dimension of the swab (12).

3. Device according to Claim 1 or 2, characterized in that the swab package (10) consists of two parts, namely a main part (22) and a lid formed as a sliding sleeve (30).

## Revendications

1. Système pour le stockage de tampons chirurgicaux avec un certain nombre de logements séparés, avantageusement agencés en plusieurs rangées, recevant chacun un tampon chirurgical, caractérisé en ce que
il s'agit de tampons chirurgicaux (12) et pour le système, d'un emballage de tampons (10),
en ce que, parmi les logements séparés, chacun est configuré en tant que logement (14) de réception d'un seul tampon et est rempli d'un seul tampon (12),
et en ce que l'emballage de tampons rempli et stérilisé (10) est enfermé dans une enveloppe en plastique.

2. Système selon la revendication 1 , caractérisé en ce que les logements (14, 24) pour un seul tampon sont en forme de cavité et le diamètre ou la grande diagonale de la cavité de réception est plus grand que la plus grande longueur du tampon (12) à y placer et la profondeur est plus grande que la plus petite dimension transversale du tampon (12).

3. Système selon la revendication 1 ou 2, caractérisé en ce que l'emballage (10) des tampons se compose de deux parties formées d'une partie principale (22) d'un manchon coulissant (30).
